Europäisches Patentamt

European Patent Office

Office européen des brevets

(11) Veröffentlichungsnummer: **0 091 647**

**A1**

(12) # EUROPÄISCHE PATENTANMELDUNG

(21) Anmeldenummer: 83103330.3

(22) Anmeldetag: 06.04.83

(51) Int. Cl.³: **A 61 F 9/06**
**A 62 B 18/08**

(30) Priorität: 10.04.82 DE 8210224 U

(43) Veröffentlichungstag der Anmeldung:
19.10.83 Patentblatt 83/42

(84) Benannte Vertragsstaaten:
AT CH DE FR GB LI SE

(71) Anmelder: Nukem GmbH
Rodenbacher Chaussee 6 Postfach 11 00 80
D-6450 Hanau 11(DE)

(72) Erfinder: Zwanzig, Dieter
Justus v. Liebig Strasse 11
D-6454 Bruchköbel(DE)

(74) Vertreter: Nowak, Gerhard
DEGUSSA AG Fachbereich Patente Postfach 1345
D-6450 Hanau 1(DE)

(54) Kombinierte Schutzmaske.

(57) Zum Arbeiten in atemgefährdenden Räumen, insbesondere in chemischen und kerntechnischen Anlagen, werden Schutzmasken benötigt, die einen Grundkörper (1), ein Filter (8) und eine Sichtscheibe besitzen. Bei der vorliegenden Schutzmaske wird an Stelle der Sichtscheibe ein auswechselbarer schachtförmiger Formkörper (3) auf den Grundkörper dicht aufgesetzt. Der Formkörper ist an der Frontseite mit einem Sichtfenster (4) und einen parallel zum Sichtfenster ausgerichteten Schweißglas (5) versehen.

82 132 KN

NUKEM GmbH
6450 Hanau 11

Kombinierte Schutzmaske

Gegenstand der Erfindung ist eine kombinierte Schutzmaske für Schweiß- und Schneidarbeiten in atemgefährdenden Räumen, insbesondere in chemischen und kerntechnischen Anlagen, im wesentlichen bestehend aus
einem Atemvollschutzmasken-Grundkörper mit Filter und
einem automatisch arbeitenden Schweißglas.

Das Schweißen und Schneiden in Räumen, die gesundheitsgefährdende Gase enthalten, oder in denen sich radioaktive Stoffe befinden, z. B. als Kontamination auf
Geräten im kerntechnischen Einsatz, erfordert Schutzvorkehrungen hinsichtlich Augenlicht und Inkorporation.
Die Schweiß- und Schneidarbeiten in derartigen Räumen
werden daher stets so ausgeführt, daß der Ausführende
zunächst eine Atemmaske mit entsprechend ausgerüstetem
Filter trägt, über die anschließend eine Schweißmaske
gestülpt ist. Der Nachteil dieser Anordnung besteht jedoch darin, daß die Gewichtsbelastung das Arbeiten erschwert und die Beweglichkeit stark eingeschränkt ist.
Nachteilig ist weiterhin, daß zwischen den Schweißvorgängen
die Schweißmaske entweder abgenommen oder zurückgeklappt wird. Auch die Verwendung von automatisch arbeitendem Glas in der Schweißmaske beseitigt die beschriebenen Nachteile nicht, da einmal dieses Glas kontaminiert bzw. angegriffen wird und das Sichtfenster der Atemmaske nicht optisch einwandfrei zu dem automatischen Glas des aufgesetzten Schweiglases ausgerichtet ist. In der DE-OS 28 11 620 ist eine Schweißschutzhaube beschrieben, die ein Filterelement an der Frontseite der Schweißerschutzhaube unterhalb des Sichtfensters enthält. Nachteilig dabei ist, daß die Augen für Kontamination enthaltende Stäube sowie für aggressive Medien völlig ungeschützt sind. Das bedeutet, daß eine solche Vorrichtung für derart gesundheitsgefährdende Räume nicht einsetzbar ist. Weiterhin wird das relativ wertvolle automatische Schweißglas beidseitig kontaminiert bzw. gegebenenfalls durch aggressive Medien angegriffen. Eine zusätzliche Brille als Augenschutz würde eine Sichtverzerrung und damit eine zusätzliche Behinderung zur Folge haben, wobei die für radioaktive oder toxische Stoffe erforderlichen verhältnismäßig großen Luftfilter weiter das Gesichtsfeld des Ausführenden und damit die Sicherheit und die Qualität der auszuführenden Arbeit beeinträchtigen würden.

Der vorliegenden Erfindung lag daher die Aufgabe zugrunde, eine kombinierte Schutzmaske für Schweiß- und Schneidarbeiten in atemgefährdenden Räumen, insbesondere in chemischen und kerntechnischen Anlagen, zu schaffen, im wesentlichen bestehend aus einem Atemvollschutzmasken-Grundkörper mit Filter und einem automatisch arbeitenden Schweißglas, die die beschriebenen Nachteile vermeidet, bequem tragbar ist und den spezifischen Belangen dieser Räume auch bei längerem Aufenthalt Rechnung trägt.

Die Aufgabe wurde neuerungsgemäß dadurch gelöst, daß auf dem Masken-Grundkörper an Stelle einer Sichtscheibe ein auswechselbarer schachtförmiger Formkörper formschlüssig dicht aufgesetzt ist, der an der Frontseite auf Augenhöhe mit einem Sichtfenster versehen ist und außerdem ein parallel zum Sichtfenster ausgerichtetes automatisch arbeitendes Schweißglas enthält. Vorzugsweise weist der Formkörper eine nach unten gerichtete Ausbuchtung auf, in die das Schweißglas eingeklemmt ist.

In interessanten Ausgestaltungen ist der Formkörper aus Blech hergestellt und gedrückt, mit einem Funkenschutz versehen und das Sichtfenster viereckig ausgebildet.

Die Erfindung gestattet einen einwandfreien Augenschutz und eine einwandfreie optische nicht verzerrende Positionierung des automatisch arbeitenden Schweißglases. Weiterhin bleibt das relativ wertvolle automatische Schweißglas sauber und kann leicht ausgewechselt werden. Der auswechselbare Formkörper kann individuell angesetzt werden, z. B. für Brillenträger usw..

Anhand der schematischen Abbildung wird die neuerungsgemäß kombinierte Schutzmaske beispielhaft näher erläutert.

Ein sichtscheibenloser Atemvollschutzmasken-Grundkörper (1) enthält ein Nasen- und Mundschutz (6) mit Rückschlagventilen, ferner Filterstutzen (7) und Filter (8). Mittels einer am Grundkörper (1) befindlichen Klemm- und Spannvorrichtung (2) ist ein schachtförmiger Formkörper (3) lösbar und austauschbar befestigt. Der Formkörper kann aus Kunststoff gefertigt sein, besonders vorteilhaft ist jedoch die Verwendung von gedrücktem Blech, z.B. von Edelstahlblech. Der Formkörper (3) enthält eine nach unten gerichtete Ausbuchtung (9), in die durch den Innenraum ein automatisch arbeitendes Schweißglas (5) mit Batterie und Schalter geführt, gehaltert und optisch fixiert ist. An der rahmenartigen Frontseite (10) des Formkörpers (3) ist ein Sichtfenster (4) befestigt, beispielsweise eine Kunstglasplatte aufgeklebt. Vorteilhafterweise ist das Sichtfenster (4) viereckig ausgebildet und seine Abmessungen etwa 100 x 110 mm. Dadurch ist ein ausreichendes Blickfeld für die voll geschützten Augen ohne arbeitsmäßige Beeinträchtigungen gegeben.

Die Tiefe des Formkörpers (3) kann den persönlichen Bedürfnissen des Trägers der neuerungsgemäßen kombinierten Schutzmaske angepaßt werden. Es ist leicht möglich, das relativ wertvolle automatische Schweißglas im Innenraum anderer neuerungsgemäßer Masken, die billig hergestellt werden können, zu positionieren und somit kostenmäßig optimal einzusetzen, zumals es stets sauber bleibt.

In manchen Anwendungsfällen ist es auch günstig, um das Sichtfenster (4) einen zusätzlichen Funkenschutz (11) zu befestigen, insbesondere aufzustecken.

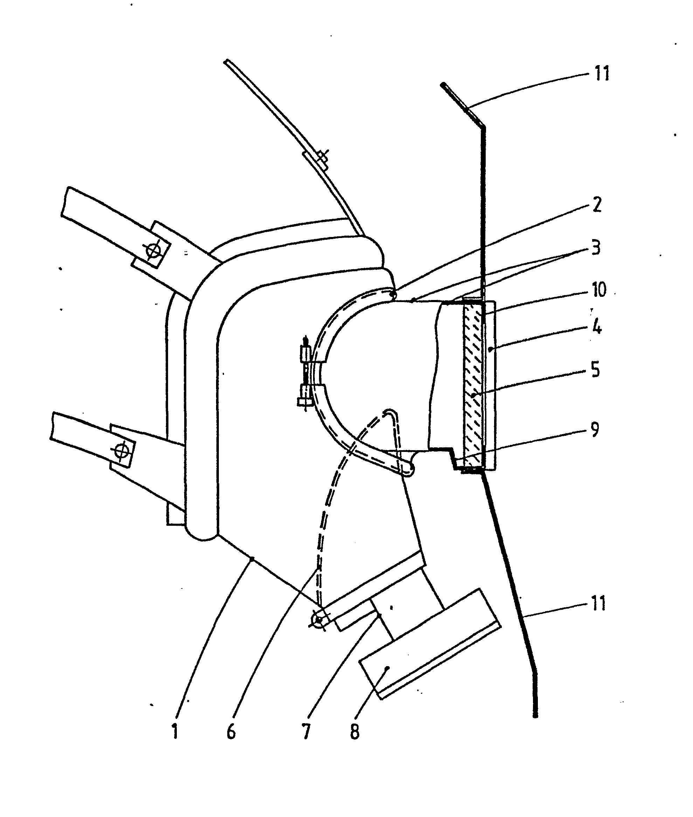

Das Ausrichten des automatisch arbeitenden Schweißglases (5) innerhalb des schachtförmigen Formkörpers (3) kann beispielsweise mittels Anschlag, Federn oder Stellschrauben (in der Abbildung nicht gezeichnet) erfolgen.

82 132 KN

NUKEM GmbH
6450 Hanau 11

**Patentansprüche**

1. Kombinierte Schutzmaske für Schweiß- und Schneidarbeiten in atemgefährdenden Räumen, insbesondere
in chemischen und kerntechnischen Anlagen, im
wesentlichen bestehend aus einem Atemvollschutzmasken-Grundkörper mit Filter und einem automatisch arbeitenden Schweißglas, <u>dadurch gekenn-
zeichnet</u>, daß auf dem Masken-Grundkörper (1) an
Stelle einer Sichtscheibe ein auswechselbarer
schachtförmiger Formkörper (3) formschlüssig dicht
aufgesetzt ist, der an der Frontseite (10) auf
Augenhöhe mit einem Sichtfenster (4) versehen ist
und außerdem ein parallel zum Sichtfenster (4)
ausgerichtetes automatisch arbeitendes Schweißglas (5) enthält.

2. Kombinierte Schutzmaske nach Anspruch 1, <u>dadurch
gekennzeichnet</u>, daß der Formkörper (3) eine nach
unten gerichtete Ausbuchtung (9) aufweist.

3. Kombinierte Schutzmaske nach Anspruch 1 und 2, dadurch gekennzeichnet, daß der Formkörper (3) aus Blech besteht.

4. Kombinierte Schutzmaske nach Anspruch 1 bis 3, dadurch gekennzeichnet, daß der aus Blech bestehende Formkörper (3) gedrückt ist.

5. Kombinierte Schutzmaske nach den Ansprüchen 1 bis 4, dadurch gekennzeichnet, daß das Sichtfenster (4) viereckig ausgebildet ist.

6. Kombinierte Schutzmaske nach den Ansprüchen 1 bis 5, dadurch gekennzeichnet, daß am Formkörper (3) ein Funkenschutz (11) befestigt ist.

7. Kombinierte Schutzmaske nach den Ansprüchen 1 bis 6, dadurch gekennzeichnet, daß der Funkenschutz (11) aufgesteckt ist.

Abb.

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-3 147 487 (HOEFTMAN)<br>* Anspruch; Figuren 1-3 *<br><br>--- | 1,5 | A 61 F 9/06<br>A 62 B 18/08 |
| A | DE-A-2 349 794 (BUDMIGER)<br>* Anspruch 1 *<br><br>--- | 1 | |
| A | DE-U-1 776 286 (DRÄGERWERK)<br><br>--- | | |
| A | US-A-2 377 122 (BAKKE)<br><br>--- | | |
| A | US-A-2 590 526 (EVANS)<br><br>--- | | |
| D,A | DE-A-2 811 620 (MORRIS et al.)<br><br>----- | | RECHERCHIERTE SACHGEBIETE (Int. Cl. ³)<br><br>A 62 B 9/00<br>A 62 B 18/00<br>A 61 F 9/00 |

Der vorliegende Recherchenbericht wurde für alle Patentansprüche erstellt.

| Recherchenort<br>BERLIN | Abschlußdatum der Recherche<br>08-06-1983 | Prüfer<br>KANAL P K |
|---|---|---|